# FASCICULE DE BREVET EUROPEEN

(11) **EP 0 814 717 B1**
(45) Date de publication et mention de la délivrance du brevet: **02.05.2002**
(21) Numéro de dépôt: 97901106.1
(22) Date de dépôt: 17.01.1997
(51) Int. Cl.: A61B 17/70

(54) **DISPOSITIF D'ANCRAGE D'OSTHEOSYNTHESE VERTEBRALE POSTERIEURE**
VERANKERUNGSVORRICHTUNG FÜR POSTERIORE VERTEBRALE OSTEOSYNTHESE
ANCHORING DEVICE FOR POSTERIOR VERTEBRAL OSTEOSYNTHESIS

(30) Priorité: 19.01.1996 FR 9600895
(43) Date de publication de la demande: 07.01.1998
(73) Titulaire: Louis, René, 13008 Marseille (FR); Louis, Christian, 13009 Marseille (FR)
(72) Inventeur: Louis, René, 13008 Marseille (FR); Louis, Christian, 13009 Marseille (FR)
(74) Mandataire: Le Forestier, Eric
(86) Numéro de dépôt international: FR9700088
(87) Numéro de publication internationale: WO9725931

(56) Documents cités:
- EP-A- 0 564 046
- FR-A- 2 695 550
- GB-A- 2 254 370
- US-A- 5 415 659

## Description

La présente invention a pour objet un dispositif d'ancrage d'ostéosynthèse vertébrale postérieure.

Le secteur technique de l'invention est le domaine de la fabrication d'implants d'ostéosynthèse destinés à la colonne vertébrale de l'homme ou de l'animal.

Lorsqu'on veut en effet stabiliser et/ou fixer deux ou plusieurs vertèbres entre elles, et/ou réduire des déformations de la colonne vertébrale (telles que cyphoses ou scolioses), il est connu d'utiliser des éléments métalliques longitudinaux, pontant plusieurs vertèbres, par exemple par plaques ou tiges, et sur lesquelles sont ancrés d'autres éléments métalliques de fixation, tels que des vis essentiellement.

Diverses demandes de brevets ont été déposées, en particulier sur différents systèmes d'ostéosynthèse vertébrale, telles que par exemple la demande N° FR 2.716.794, publiée le 8 septembre 1995 et déposée par la Société américaine SOFAMOR (Inventeur : Monsieur STEIB), sur un « Connecteur pour l'instrumentation d'ostéosynthèse rachidienne destinée à une fixation lombaire ou sacrée ou ilio-sacrée », ou la demande N° FR 2.712.486, publiée le 24 mai 1995 de Messieurs BRESLAV, CATON, etc... sur une « Prothèse intervertébrale », ou encore la demande N° FR 2.697.874, publiée le 13 mai 1994 par la Société de Fabrication de Matériel Orthopédique (Inventeur : Monsieur COTREL), intitulée : « Dispositif de liaison d'un élément longiligne et d'un support de cet élément ».

On peut également citer d'autres demandes plus anciennes, telles que la demande N° FR 2.672.202, publiée le 7 août 1992, de la Société SAFIR (Inventeurs : Monsieur GRAF et al), sur un « Implant chirurgical osseux, notamment pour stabilisateur intervertébral », ou encore la demande N° FR 2.651.992, publiée le 22 mars 1991 par la Société de Fabrication de Matériel Orthopédique (Inventeur : Monsieur LAURAIN), sur un « Implant pour ostéosynthèse rachidienne dorsolombaire antérieure, destiné à la correction de cyphoses ».

Le brevet US - 54 15 659 montre un système d'ancrage postérieur vertébral utilisant plusieurs crochets, avec six points de fixation sur une même vertèbre.

Tous ces systèmes, comme beaucoup d'autres qu'il serait trop long de reprendre ici, utilisent essentiellement pour leur fixation sur les vertèbres, des vis nécessitant un taraudage dans les parties osseuses, ce qui est assez délicat et de toute façon toujours aux dépens de la fragilité de l'os. Il existe également des systèmes de type crochets ou cerclage autour des régions lamaires, épineuses, transverses ou articulaires (mais jamais isthmiques) de chaque vertèbre, mais ces régions considérées assez fragiles n'autorisent pas seules d'efforts trop importants de couples de redressement.

Le problème posé est donc de pouvoir réaliser des systèmes d'ostéosynthèse vertébrale, en utilisant des dispositifs d'ancrage non vissés dans les vertèbres, mais suffisamment solidaires de celles-ci pour permettre des couples de redressement importants, sans fragiliser, ni risquer de détérioration desdites vertèbres.

Une solution au problème posé est un dispositif d'ancrage d'ostéosynthèse vertébrale postérieure, comportant au moins quatre points de fixation constitués chacun d'un crochet, lesquels crochets étant opposés deux à deux suivant deux axes perpendiculaires XX'/ZZ' et chaque paire de crochets ainsi constituée formant une pince, l'une considérée suivant l'axe XX' latéral et dite isthmique, et l'autre considérée axiale suivant l'axe ZZ' dite laminaire ; lesdits crochets sont solidaires de supports, qui les relient à un même corps central de préférence monobloc et les extrémités de ces crochets sont d'une largeur d'au moins 5 millimètres.

Le corps central reçoit un moyen de fixation immobilisant sur lui-même tout moyen de liaison avec au moins un autre dispositif d'ancrage, tel qu'au moins une tige ; le moyen de fixation de celle-ci peut être alors une bride la chevauchant et l'immobilisant sur ledit corps central par serrage.

Dans un mode de réalisation préférentiel, chaque support de chacun desdits crochets de chaque paire définie ci-dessus, est monté coulissant par rapport audit corps central et réglable suivant les axes ZZ'/XX', permettant leur rapprochement et leur éloignement réciproque, lesdits supports de crochets coulissants dans ledit corps central pouvant être immobilisés dans celui-ci par tout moyen traversant ledit corps central, tel que par exemple des vis de blocage.

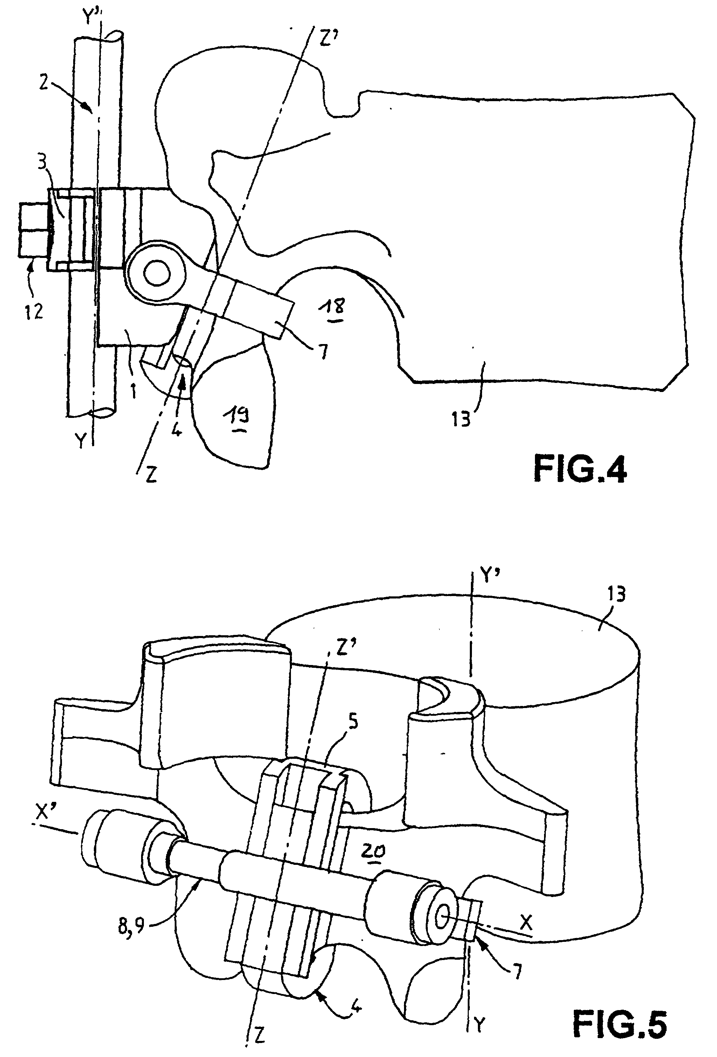

Un tel système répond bien au problème posé en permettant en effet un ancrage sur chaque arc postérieur de la vertèbre, comme représenté sur les figures 4 et 5 : deux desdits crochets dits isthmiques tels que définis ci-dessus, opposés latéralement suivant l'axe XX' horizontal du dispositif une fois en place, quand la personne concernée est en position debout, peuvent s'accrocher sur les régions isthmiques droite et gauche de la vertèbre à la partie postérieure des foramens, qui sont les trous de conjugaison par où sortent les nerfs rachidiens, ce qui n'avait jamais été fait jusqu'à présent ; les deux autres crochets opposés axialement et disposés suivant un axe perpendiculaire ZZ', donc vertical une fois en place, s'accrochent sur le milieu des bords supérieur et inférieur des lames de la même vertèbre ; ces ensembles de crochets forment ainsi deux pinces perpendiculaires, solidarisées par une pièce commune au croisement de leurs supports.

Dans un mode particulier de réalisation, les supports des crochets dits isthmiques disposés latéralement sur les régions isthmiques, sont constitués par deux arbres cylindriques concentriques coulissant l'un dans l'autre, de section circulaire et permettant la rotation desdits crochets suivant leur axe de serrage, assurant ainsi une adaptation parfaite de l'ancrage avec la morphologie de la vertèbre.

Les supports des crochets dits laminaires disposés axialement ou verticalement pour s'accrocher sur les bords supérieur et inférieur des lames de la même vertèbre, peuvent être constitués de préférence par deux guides cylindriques mais non circulaires coulissant et s'emboîtant l'un dans l'autre, et coopérant avec un rail du corps central, interdisant alors leur rotation, par rapport à leur axe de serrage, afin de bien immobiliser ledit corps central sur la vertèbre.

Les supports coulissants de chaque crochet permettent d'adapter la longueur de la pince qu'ils constituent à la dimension de la vertèbre sur laquelle on veut s'ancrer, sans nécessiter d'intervention sur l'os de la vertèbre et donc en évitant toute fragilisation de celui-ci, tout en assurant par le tétrapode ainsi constitué, un parfait ancrage permettant des efforts de traction ou de torsion importants.

Les crochets existant à ce jour, tels qu'évoqués précédemment, sont toujours étroits tels que pointus ou arrondis à leur extrémité et ne prennent en effet jamais appui sur les isthmes verticaux, ni ne sont combinés avec d'autres crochets disposés sur la ligne médiane des lames vertébrales inférieure et supérieure.

Le résultat est un nouveau dispositif d'ancrage d'ostéosynthèse vertébrale postérieure, constituant la présente invention, dont on pourrait citer d'autres avantages que ceux cités ci-dessus mais qui en montrent déjà suffisamment pour en prouver la nouveauté et l'intérêt.

La description et les figures ci-après représentent un exemple de réalisation de l'invention, mais n'ont aucun caractère limitatif : d'autres réalisations sont possibles, dans le cadre de la portée et de l'étendue des revendications, en particulier en changeant la forme des différents supports de crochets et du corps central supportant lesdits supports et les moyens de liaison entre dispositifs d'ancrage successifs.

La figure 1 représente un dispositif suivant l'invention, en vue perspective éclatée, chaque élément étant démonté.

La figure 2 est une vue perspective du même dispositif que la figure 1, mais une fois monté.

La figure 3 est une vue d'un des dispositifs des figures précédentes en vue crânienne, donc de dessus une fois mis en place.

La figure 4 est une vue latérale droite du même dispositif ancré sur une vertèbre.

La figure 5 est une vue schématique simplifiée des seuls crochets et de leur support mis en place suivant la figure 4.

Le dispositif d'ancrage d'ostéosynthèse vertébrale postérieure sur une vertèbre 13, comporte suivant l'invention au moins quatre points de fixation constitués chacun d'un crochet 4, 5, 6, 7, lesquels crochets étant opposés deux à deux suivant deux axes perpendiculaires XX' horizontal une fois mis en place en considérant la personne concernée en position debout, et ZZ' vertical, et chaque paire ainsi constituée formant une pince.

Les extrémités desdits crochets 4, 5, 6 et 7 sont suffisamment larges pour assurer une répartition de l'appui et de la force de serrage sur les parties de la vertèbre, tout en empêchant toute rotation du dispositif, limitant ainsi ses déplacements éventuels par rapport à ladite vertèbre sur laquelle il est ancré.

Pour cela, la largeur des extrémités desdits crochets peut être par exemple d'au moins 5 millimètres au niveau de leur surface d'appui sur les parties des vertèbres recevant lesdits crochets.

Comme indiqué précédemment, ceux-ci sont solidaires de supports qui les relient à un même corps central 1, de préférence monobloc, lesquels supports coulissent et sont réglables par rapport à celui-ci suivant les axes respectivement ZZ' pour la paire verticale 4, 5 et XX' pour la paire horizontale 6, 7 permettant le rapprochement desdits crochets et/ou leur éloignement réciproque.

En ce qui concerne les crochets latéraux 6, 7 dits isthmiques, leurs supports peuvent être constitués de deux arbres cylindriques et concentriques 8, 9, dont l'un 9 peut être creux pour permettre l'introduction de l'autre arbre 8 mâle, coulissant alors l'un dans et par rapport à l'autre, jusqu'à venir bloquer par leurs extrémités respectives en forme de têtes, lesdits crochets latéraux 6, 7 sur l'extérieur des régions isthmiques 19 à la partie postérieure des foramens 18 de la vertèbre 13 ; la section circulaire desdits arbres permet leur rotation et/ou celle des crochets 6, 7 pour s'adapter à toute orientation angulaire des régions isthmiques par rapport à l'axe ZZ' du dispositif et/ou YY' de la ligne de la colonne vertébrale que l'on veut redresser.

Par contre, les supports coulissants 15, 16 des crochets, l'un dit supralaminaire 5 à la partie supérieure et l'autre dit infralaminaire 4 à la partie inférieure, disposés suivant l'axe vertical ZZ' correspondant aux lames 20 de la même vertèbre 13, sont constitués au moins par deux guides cylindriques mais non circulaires coulissant et s'emboîtant l'un dans l'autre, coopérant avec un rail 17 du corps central 1, interdisant la rotation desdits supports par rapport à l'axe ZZ', comme représenté sur les figures jointes : le support 15 du crochet 5 y est par exemple de section en forme de U, à l'intérieur duquel le support 16 du crochet 4 de section rectangulaire peut alors coulisser, l'ensemble étant maintenu dans un guide 17 de section intérieure correspondante à la forme extérieure également rectangulaire du support 15, dans le corps monobloc central 1.

L'ensemble desdits supports, au nombre de quatre, suivant le présent mode de réalisation représenté sur les figures, coulisse donc dans ledit corps central 1, où ils sont immobilisés par tout moyen traversant celui-ci, tel que par exemple des vis de blocage 10, 11 taraudées dans le corps central 1 et dont les extrémités viennent porter sur lesdits supports respectifs qu'elles servent à bloquer.

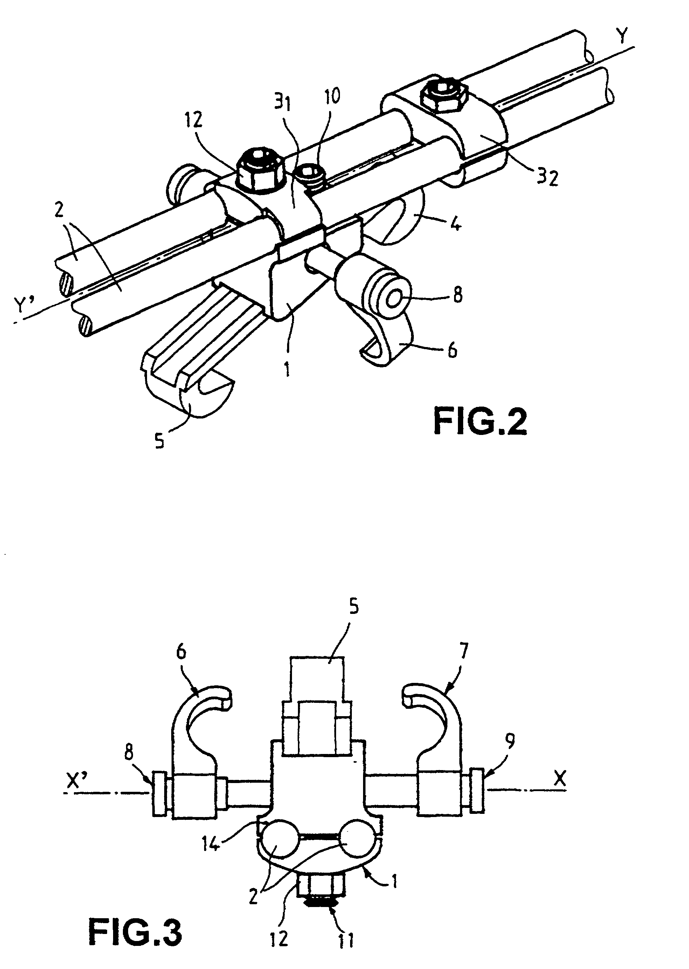

La pièce commune ou corps monobloc central 1 comporte près de sa ligne médiane supérieure par rapport aux crochets, au moins une gouttière 14 permettant d'y loger et d'y bloquer tout moyen de liaison, tel qu'une tige 2 métallique longitudinale, avec un autre dispositif d'ancrage 3₂ tel que représenté sur la figure 2 par son seul moyen de fixation dudit dispositif sur lesdites tiges 2.

De préférence, ledit moyen de liaison est donc constitué par deux tiges 2 métalliques et le moyen de fixation de celles-ci sur ledit corps central 1 alors dans les deux gouttières 14, est une bride 3 chevauchant lesdites tiges et immobilisant celles-ci sur ledit corps central par serrage, lequel serrage peut être réalisé par un écrou 12 vissé sur l'un desdits moyens d'immobilisation des supports de crochets, tels qu'une des vis de blocage 10, 11.

Les deux tiges métalliques 2 longitudinales sont de longueur adaptée à l'étendue de la colonne vertébrale que l'on veut traiter suivant le montage souhaité.

Un système d'instruments ancillaire permet alors d'écarter ou de rapprocher les tétrapodes ou doubles pinces ainsi constituées et fixées sur deux ou plusieurs vertèbres pour allonger ou raccourcir une courbure vertébrale, leur fixation par serrage en quatre points permettant un tel effort sans risque.

Des tiges provisoires peuvent être fixées sur les crochets isthmiques permettant d'exercer un effet de dérotation sur les vertèbres 13 et les deux tiges paramédianes 2 axiales postérieures, précourbées suivant la forme finale souhaitée du rachis, servent à bloquer les tétrapodes par leur bride 3 de fixation et d'immobilisation, après réduction des déformations.

Le montage peut s'accompagner de greffes osseuses sans obligation d'ôter ultérieurement ledit système, mais un tel démontage est de toute façon facile à réaliser par dévissage des différents systèmes de blocage 12, 10, 11 et démontage de l'ensemble des pièces qui coulissent les unes par rapport aux autres, et sont donc faciles à enlever.

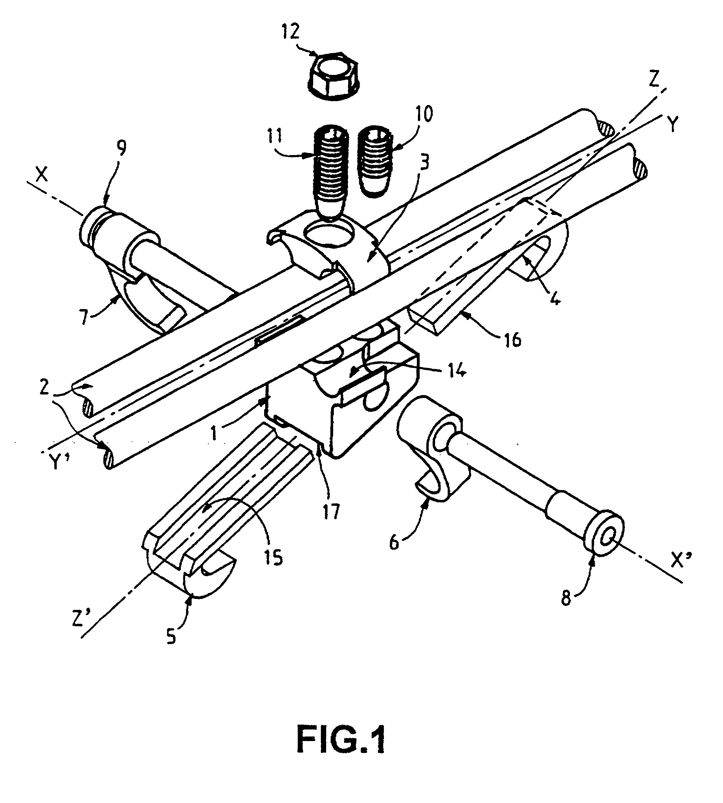

On remarquera que, sur les figures 4 et 5, du fait de la morphologie des vertèbres, les axes ZZ' des crochets dits verticaux ou laminaires 4, 5 et YY' du moyen de liaison 2, correspondant en fait à celui de l'axe de la vertèbre 13, forment un angle ouvert vers le haut, ZZ' s'écartant de YY' dans le sens des aiguilles d'une montre ; alors que sur les figures 1 et 2, le dispositif est représenté en fait à l'envers, sa partie normalement haute étant dirigée vers le bas.

## Revendications

1. Dispositif d'ancrage d'ostéosynthèse vertébrale postérieure, comportant au moins quatre points de fixation, constitués chacun d'un crochet (4, 5, 6, 7), **caractérisé en ce que** lesdits crochets sont opposés deux à deux suivant deux axes perpendiculaires XX'/ZZ' et chaque paire de crochets ainsi constituée forme une pince, l'une latérale considérée suivant l'axe XX' dite isthmique, et l'autre axiale ZZ' dite laminaire, et lesdits crochets sont solidaires de supports qui les relient suivant lesdits axes XX' et ZZ' à un même corps (1) central.

2. Dispositif d'ancrage suivant la revendication 1, **caractérisé en ce que** ledit corps central (1) reçoit un moyen de fixation (3) immobilisant sur ledit corps central tout moyen de liaison avec au moins un autre dispositif d'ancrage.

3. Dispositif d'ancrage suivant la revendication 2, **caractérisé en ce que** chaque support de chacun desdits crochets de chaque paire (4, 5) et (6, 7) est monté coulissant par rapport audit corps central (1) et réglable suivant leur axe respectivement ZZ'/XX' permettant leur rapprochement et leur éloignement réciproque.

4. Dispositif d'ancrage suivant la revendication 3, **caractérisé en ce que** lesdits supports de crochets coulissant dans ledit corps central (1) sont immobilisés dans celui-ci par tout moyen traversant ledit corps central.

5. Dispositif d'ancrage suivant la revendication 4, **caractérisé en ce que** les moyens d'immobilisation des supports de crochets dans ledit corps central (1), sont des vis de blocage (10, 11).

6. Dispositif d'ancrage suivant la revendication 2, **caractérisé en ce que** ledit moyen de liaison est constitué par au moins une tige (2) et son moyen de fixation (3) est une bride chevauchant ladite tige (2), et immobilisant celle-ci sur ledit corps central (1) par serrage.

7. Dispositif d'ancrage suivant la revendication 6, **caractérisé en ce que** le serrage de ladite bride (3) de fixation de la tige (2) de liaison, est réalisé par un écrou (12) vissé sur l'un des moyens (10, 11) d'immobilisation des supports de crochets.

8. Dispositif d'ancrage suivant l'une quelconque des revendications 2 à 7, **caractérisé en ce que** les supports des crochets (6, 7) disposés latéralement, dits isthmiques, sont constitués par deux arbres (8, 9) cylindriques, concentriques, coulissant l'un dans l'autre, de section circulaire et permettant la rotation des crochets (6, 7) par rapport au corps central (1) monobloc suivant l'axe XX'.

9. Dispositif d'ancrage suivant l'une quelconque des revendications 2 à 8, **caractérisé en ce que** les supports des crochets (4, 5) disposés axialement dits laminaires, sont constitués par deux guides (16, 15) cylindriques, coulissant et s'emboîtant l'un dans l'autre, et coopérant avec un rail (17) interdisant leur rotation par rapport au corps central (1) suivant l'axe ZZ'.

10. Dispositif d'ancrage suivant l'une quelconque des revendications 1 à 9 **caractérisé en ce que** les deux dits crochets (6, 7) sont opposés latéralement suivant l'axe XX' dits isthmiques, pour s'accrocher à la partie postérieure des foramens (18) droit et gauche de la même vertèbre (13).

11. Dispositif d'ancrage suivant l'une quelconque des revendications 1 à 10, **caractérisé en ce que** les deux dits crochets (4, 5) sont opposés axialement suivant l'axe ZZ' dits laminaires, pour s'accrocher sur le milieu des bords supérieur et inférieur des lames (20) de la même vertèbre (13).

## Patentansprüche

1. Verankerungsvorrichtung zur rückseitigen Osteosynthese von Wirbeln, mit mindestens vier Befestigungspunkten, die jeder von einem Haken (4, 5, 6, 7) gebildet sind, **dadurch gekennzeichnet, daß** die genannten Haken einander paarweise gemäß zweier senkrechter Achsen XX'/ZZ' gegenüberliegen und jedes so gebildete Paar von Haken eine Zange bildet, wobei die eine, seitliche, gemäß der Achse XX' betrachtete isthmisch genannt ist und die andere, axiale ZZ' laminar genannt ist, und daß die genannten Haken fest mit Trägern verbunden sind, die sie gemäß der genannten Achsen XX' und ZZ' mit ein und demselben zentralen Körper (1) verbinden.

2. Verankerungsvorrichtung nach Anspruch 1, **dadurch gekennzeichnet, daß** der genannte, zentrale Körper (1) ein Befestigungsmittel (3) aufnimmt, das auf dem genannten, zentralen Körper jedes Verbindungsmittel mit mindestens einer anderen Verankerungsvorrichtung festlegt.

3. Verankerungsvorrichtung nach Anspruch 2, **dadurch gekennzeichnet, daß** jeder Träger eines jeden der genannten Haken eines jeden Paares (4, 5) und (6, 7) in Bezug auf den genannten, zentralen Körper (1) verschieblich und in seiner jeweiligen Achse ZZ'/XX' einstellbar angebracht ist, was ihre wechselseitige Annäherung und Entfernung gestattet.

4. Verankerungsvorrichtung nach Anspruch 3, **dadurch gekennzeichnet, daß** die genannten Träger der Haken, die im genannten, zentralen Körper (1) verschieblich sind, in diesem durch jedes Mittel festgelegt sind, das den genannten, zentralen Körper durchsetzt.

5. Verankerungsvorrichtung nach Anspruch 4, **dadurch gekennzeichnet, daß** die Mittel zur Festlegung der Träger der Haken im genannten, zentralen Körper (1) Klemmschrauben (10, 11) sind.

6. Verankerungsvorrichtung nach Anspruch 2, **dadurch gekennzeichnet, daß** das genannte Verbindungsmittel von mindestens einer Stange (2) gebildet ist, und daß ihr Befestigungsmittel (3) eine Rohrschelle ist, die die genannte Stange (2) überdeckt und diese auf dem genannten, zentralen Körper (1) durch Klemmung festlegt.

7. Verankerungsvorrichtung nach Anspruch 6, **dadurch gekennzeichnet, daß** die Klemmung der genannten Rohrschelle (3) zur Befestigung der Verbindungsstange (2) durch eine Mutter (12) hergestellt ist, die auf einen der Mittel (10, 11) zur Festlegung der Träger der Haken aufgeschraubt ist.

8. Verankerungsvorrichtung nach irgendeinem der Ansprüche 2 bis 7, **dadurch gekennzeichnet, daß** die Träger der Haken (6, 7), die seitlich angeordnet sind und isthmisch genannt sind, von zwei zylindrischen, konzentrischen Achsen (8, 9) gebildet sind, die ineinander verschieblich sind, einen kreisförmigen Querschnitt aufweisen und die Drehung der Haken (6, 7) in Bezug auf den zentralen, einstückigen Körper (1) gemäß der Achse XX' gestatten.

9. Verankerungsvorrichtung nach irgendeinem der Ansprüche 2 bis 8, **dadurch gekennzeichnet, daß** die Träger der Haken (4, 5), die axial angeordnet und laminar genannt sind, von zwei zylindrischen Führungen (16, 15) gebildet sind, die verschieblich sind und ineinandersitzen, und daß sie mit einer Schiene (17) zusammenwirken, die ihre Drehung in Bezug auf den mittigen Körper (1) gemäß der Achse ZZ' untersagt.

10. Verankerungsvorrichtung nach irgendeinem der Ansprüche 1 bis 9, **dadurch gekennzeichnet, daß** die beiden, genannten Haken (6, 7), die isthmisch genannt sind, seitlich gemäß der Achse XX' gegenüberliegen, um sich am hinteren Teil der Öffnung (18) rechts und links desselben Wirbels (13) zu verhaken.

11. Verankerungsvorrichtung nach irgendeinem der Ansprüche 1 bis 10, **dadurch gekennzeichnet, daß** die beiden, genannten Haken (4, 5), die laminar genannt sind, axial gemäß der Achse ZZ' gegenüberliegen, um sich in der Mitte des oberen und unteren Randes der Lamellen (20) desselben Wirbels (13) zu verhaken.

## Claims

1. Anchoring device for posterior vertebral osteosynthesis, comprising at least four fixation points, each consisting of a hook (4, 5, 6, 7), **characterized in that** said hooks are arranged opposite one another in two pairs along two perpendicular axes XX'/ZZ' and each pair of hooks thus constituted forms a gripping assembly, of which one is lateral considered along the axis XX' referred to as isthmic, and the other is axial ZZ' and referred to as laminar, and said hooks are integral with supports which connect them along said axes XX' and ZZ' to one and the same central body (1).

2. Anchoring device according to Claim 1, **characterized in that** said central body (1) receives a fixation means (3) immobilizing, on said central body, any means of connection to at least one other anchoring device.

3. Anchoring device according to Claim 2, **characterized in that** each support of each of said hooks of each pair (4, 5) and (6, 7) is slidably mounted with respect to said central body (1) and is adjustable along its respective axis ZZ'/XX', allowing them to be moved closer together or moved further apart.

4. Anchoring device according to Claim 3, **characterized in that** said hook supports sliding in said central body (1) are immobilized in the latter by any means passing through said central body.

5. Anchoring device according to Claim 4, **characterized in that** the means of immobilizing the hook supports in said central body (1) are locking screws (10, 11).

6. Anchoring device according to Claim 2, **characterized in that** said means of connection is formed by at least one rod (2), and its fixation means (3) is a bracket which straddles said rod (2) and immobilizes the latter on said central body (1) by clamping.

7. Anchoring device according to Claim 6, **characterized in that** the clamping of said bracket (3) for fixing the connection rod (2) is achieved by a nut (12) which is screwed onto one of the means (10, 11) of immobilizing the hook supports.

8. Anchoring device according to any one of Claims 2 to 7, **characterized in that** the supports for the hooks (6, 7) arranged laterally, referred to as isthmic, are formed by two concentric cylindrical shafts (8, 9) sliding one inside the other, of circular cross section and permitting the rotation of the hooks (6, 7) with respect to the single-piece central body (1) along the axis XX'.

9. Anchoring device according to any one of Claims 2 to 8, **characterized in that** the supports for the hooks (4, 5) arranged axially, referred to as laminar, are formed by two cylindrical guides (16, 15) which slide and engage one inside the other and which cooperate with a rail (17) which prohibits their rotation with respect to the central body (1) along the axis ZZ'.

10. Anchoring device according to any one of Claims 1 to 9, **characterized in that** the two said hooks (6, 7) are laterally opposed along the axis XX' and referred to as isthmic, so as to hook onto the posterior part of the right and left foramina (18) of the same vertebra (13).

11. Anchoring device according to any one of Claims 1 to 10, **characterized in that** the two said hooks (4, 5) are axially opposed along the axis ZZ' and referred to as laminar, so as to hook onto the centre of the upper and lower margins of the laminas (20) of the same vertebra (13).
